# FASCICULE DE BREVET EUROPEEN

(11) **EP 2 371 310 B1**
(45) Date de publication et mention de la délivrance du brevet: **05.11.2014**
(21) Numéro de dépôt: 11305320.1
(22) Date de dépôt: 22.03.2011
(51) Int. Cl.: A61F 2/28, A61F 2/38, A61F 2/30, A61F 2/48

(54) **Dispositif de déplacement de tissus, notamment de tissus osseux**
Vorrichtung zur Verschiebung von Gewebe, insbesondere Knochengewebe
Device for displacement of tissue, in particular bone tissue

(30) Priorité: 23.03.2010 FR 1001127
(43) Date de publication de la demande: 05.10.2011
(73) Titulaire: Soubeiran, Arnaud, 75014 Paris (FR)
(72) Inventeur: Soubeiran, Arnaud, 75014 Paris (FR)
(74) Mandataire: Demulsant, Xavier

(56) Documents cités:
- WO-A1-01/78614
- FR-A1- 2 901 991
- US-A- 5 704 939

## Description

L'invention concerne les dispositifs de déplacement de tissus à l'intérieur de l'organisme, notamment de tissus osseux, dont la géométrie peut être modifiée progressivement et réversiblement sans ré-opération, ni anesthésie, et plus particulièrement ceux utilisant une tige filetée montée de manière à ce qu'elle ne travaille qu'en traction et que sa longueur chargée diminue quand le dispositif s'allonge.

De tels dispositifs sont décrits dans les documents WO2007144489 et FR200904306 du présent inventeur. Les prothèses osseuses à croissance, les clous d'allongement et de transport osseux et les tiges pour la correction du thorax et du rachis réalisés suivant ces documents donnent de bons résultats cliniques.

D'autres dispositifs de déplacement de tissus à l'intérieur de l'organisme, notamment de tissus osseux, dont la géométrie peut être modifiée réversiblement sans ré-opération ni anesthésie utilisent une tige filetée mais qui travaille en compression et dont la longueur chargée augmente quand le dispositif s'allonge avec un risque de flambage croissant et avec la nécessité pour obtenir des efforts suffisants pour entraîner la vis en rotation d'utiliser un système réducteur consommateur d'espace et couteux comme décrits notamment dans les documents US6849076, US20070270803 ou WO2009058546.

Nous avons trouvé une autre disposition qui conserve les avantages des dispositions connues dans lesquelles la tige filetée est montée de manière à ce qu'elle ne travaille qu'en traction et que sa longueur chargée diminue quand le dispositif s'allonge tout en permettant un potentiel de croissance plus grand pour une longueur de dispositif donnée et une résistance mécanique à la flexion améliorée.

Les dispositifs de déplacement de tissus à l'intérieur de l'organisme, notamment de tissus osseux, selon l'invention, comprennent une pièce de référence, une pièce de transport, un écrou monté sur la tige filetée, un arbre de commande et des moyens d'entraînement reliant l'arbre de commande à l'écrou, la pièce de transport étant montée coulissante par rapport à la pièce de référence et limitée en rotation par rapport à la pièce de référence par une première butée qui coopère avec un premier appui, la rotation de la tige filetée par rapport à la pièce de référence étant bloquée par la première butée, l'écrou étant libre de tourner par rapport à la pièce de référence.

Cette disposition permet de ramener les frottements répartis sur deux sites, l'écrou et le contact butée/appui, dans les dispositifs connus au seul écrou ce qui permet un fonctionnement plus fluide du dispositif : dans les dispositions connues, l'écrou peut tourner sans que le contact butée/appui ait bougé et vice-versa, la vis subissant alors une torsion entre ces deux sites de frottements, ce qui induit un hystérésis entre l'angle de rotation de l'arbre d'entrée et l'allongement effectif du dispositif, ainsi qu'un fonctionnement par à-coups. Dans le dispositif suivant l'invention, l'écrou tourne si tous les frottements ont été vaincus et entraîne directement une variation de longueur du dispositif.

Avantageusement, la première butée est solidaire d'une extrémité de la tige filetée, le premier appui étant solidaire d'une extrémité de la pièce de référence, la première butée et l'écrou étant disposés de part et d'autre du premier appui, la translation longitudinale de la tige filetée par rapport à la pièce de référence dans le sens première butée vers premier appui étant limitée par la coopération de la première butée avec le premier appui.

Avantageusement, le dispositif comporte une seconde butée solidaire d'une extrémité de la tige filetée et apte à coopérer avec un second appui solidaire de la pièce de référence pour limiter la translation longitudinale de la tige filetée par rapport à la pièce de référence dans le sens premier appui vers première butée, lesdits premier et second appuis étant placés entre lesdites première et seconde butées et ledit écrou étant apte à se déplacer le long de la tige filetée entre lesdits premier et second appuis.

En effet, dans le dispositif de déplacement de tissus à l'intérieur de l'organisme, notamment de tissus osseux, suivant l'invention qui comprend une pièce de référence avec une première et une seconde extrémité, une pièce de transport avec une première et une seconde extrémité et montée coulissante par rapport à la pièce de référence, une tige filetée avec une première et une seconde extrémité, les premières extrémités de la pièce de transport et de la tige filetée étant constamment du coté de la première extrémité de la pièce de référence par rapport à sa seconde extrémité, une première butée solidaire de la seconde extrémité de la tige filetée et qui coopère avec un premier appui solidaire de la seconde extrémité de la pièce de référence pour limiter la translation longitudinale de la tige filetée par rapport à la pièce de référence dans le sens première butée vers premier appui, un écrou monté sur la tige filetée, la première butée et l'écrou étant disposés de part et d'autre du premier appui, des moyens pour transformer le déplacement dans un premier sens de l'écrou le long de la tige filetée en déplacement de la pièce de transport par rapport à la pièce de référence et un arbre de commande:
- la première butée coopère aussi avec le premier appui pour également bloquer la rotation de la tige filetée par rapport à la pièce de référence,
- l'écrou est libre de tourner par rapport à la pièce de référence et,
- des moyens d'entraînement relient l'arbre de commande à l'écrou.

Avantageusement, le dispositif suivant l'invention peut aussi comporter :
- une seconde butée solidaire de la première extrémité de la tige filetée et apte à coopérer avec un second appui solidaire de la pièce de référence pour limiter la translation longitudinale de la tige filetée par rapport à la pièce de référence dans le sens premier appui vers première butée lesdits premier et second appuis étant placés entre lesdites première et seconde butées et ledit écrou étant apte à se déplacer le long de la tige filetée entre lesdits premier et second appuis.
- des moyens pour transformer le déplacement dans le second sens, opposé au premier sens, de l'écrou le long de la tige filetée en déplacement de la pièce de transport par rapport à la pièce de référence.

Suivant diverses formes de réalisation de l'invention :
- l'arbre de commande comprend un aimant permanent.
- l'aimant permanent est un aimant néodyme à aimantation sensiblement diamétrale qui comporte un perçage axial pour laisser passer notamment la tige filetée et les moyens d'entraînement qui relient l'arbre de commande à l'écrou sont une enveloppe rigide solidaire de l'écrou et dans laquelle est maintenu l'aimant permanent.
- les moyens pour transformer le déplacement dans un premier sens de l'écrou le long de la tige filetée en déplacement de la pièce de transport par rapport à la pièce de référence sont une première surface d'appui solidaire de l'écrou et une seconde surface d'appui solidaire de la pièce de transport, aptes à coopérer et sensiblement perpendiculaires à l'axe de la tige filetée.
- les moyens pour transformer le déplacement dans un second sens de l'écrou le long de la tige filetée en déplacement de la pièce de transport par rapport à la pièce de référence sont une troisième surface d'appui solidaire de l'écrou et une quatrième surface d'appui solidaire de la pièce de transport, aptes à coopérer et sensiblement perpendiculaires à l'axe de la tige filetée.
- la première butée coopère également avec des surfaces de la pièce de transport pour assurer le guidage en rotation et la limitation en translation de cette dernière par rapport à la pièce de référence.

La structure et les avantages du dispositif de déplacement de tissus à l'intérieur de l'organisme, notamment de tissus osseux, suivant la présente invention seront mieux compris à la lumière de la description de modes de réalisations préférés, faite ci-après notamment en référence aux dessins annexés dans lesquels :
- la figure 1 est une vue éclatée en perspective et dans lesquelles deux pièces ont été partiellement écorchées d'un dispositif selon un premier mode de réalisation;
- la figure 2 est une vue en coupe du dispositif de la figure 1 assemblé et avant allongement;
- la figure 3 est une vue en coupe du dispositif de la figure 1 assemblé et après allongement partiel;
- la figure 4 est une vue en perspective d'un dispositif selon un second mode de réalisation;
- la figure 5 est une vue en coupe du dispositif de la figure 4 assemblé et avant allongement;
- la figure 6 est une vue éclatée en perspective d'un dispositif selon un troisième mode de réalisation ;
- la figure 7 est une vue en coupe du dispositif de la figure 6 assemblé et avant allongement ;
- la figure 8 est une vue en coupe du dispositif de la figure 6 assemblé et après allongement partiel.

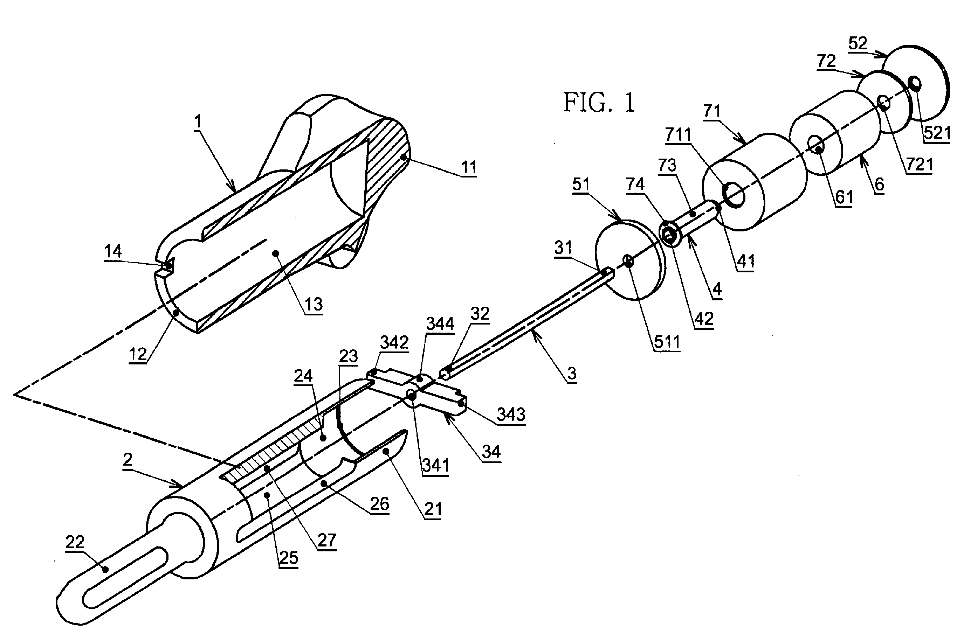

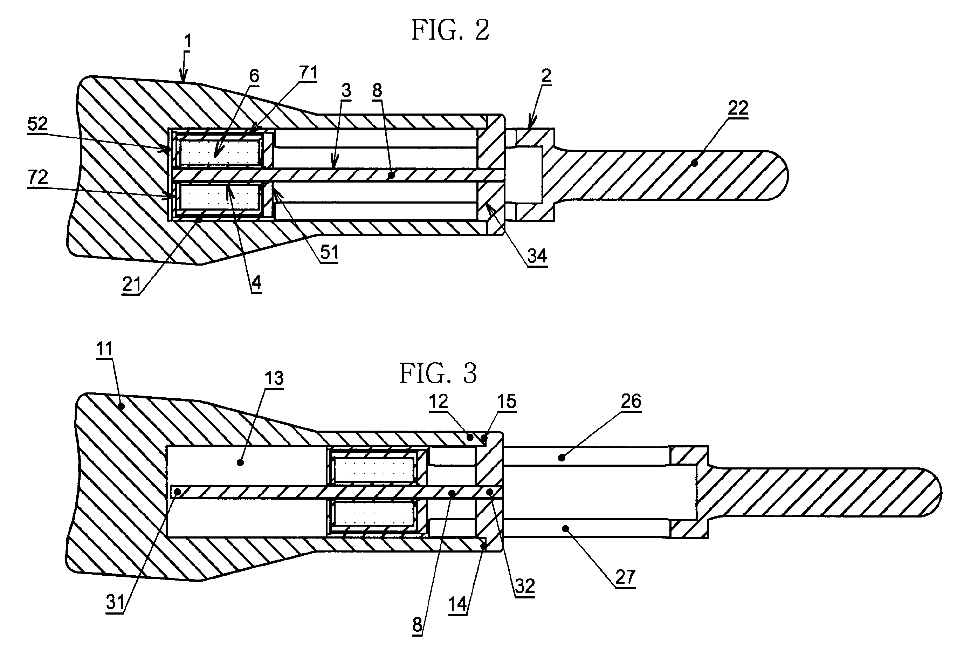

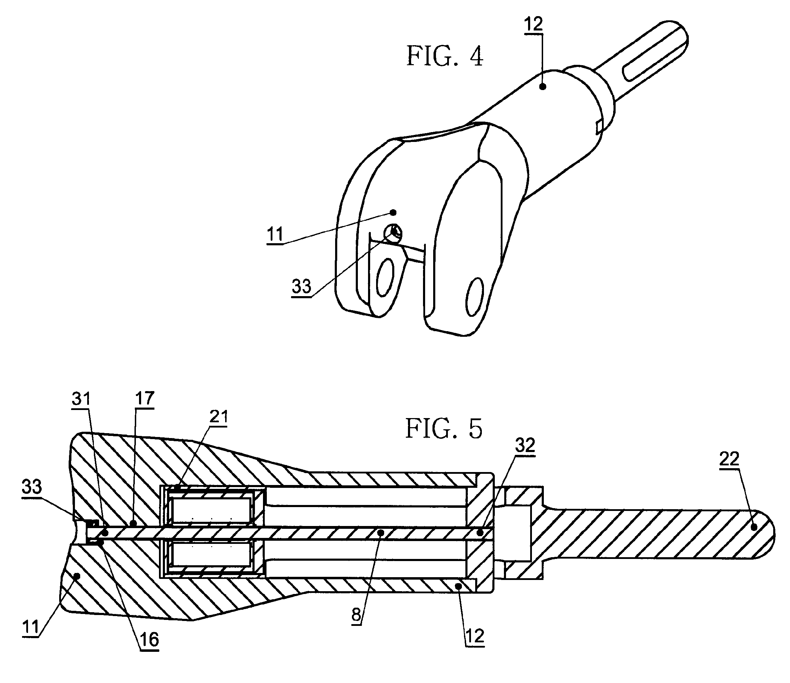

On précise que si une référence n'est pas indiquée sur une figure elle peut être facilement retrouvée sur une autre. En particulier, pour une meilleure lisibilité, les éléments déjà référencés sur les figures 1 à 3 et repris sur les figures 4 et 5 n'y sont pas tous référencés.

On se réfère maintenant aux figures 1 à 3 qui représentent un premier mode de réalisation dans une forme de réalisation bien adaptée à la réalisation de prothèses de croissance. Pour cet exemple démonstratif mais nullement limitatif de ce premier mode de réalisation, c'est une prothèse de fémur distal à croissance qui est représentée et décrite, elle comprend:
- une pièce de référence 1 avec
   o une première extrémité 11, distale, extérieurement en forme connue de fémur distal prothétique à charnière et apte à coopérer avec un axe et une pièce tibiale non représentés,
   o et une seconde extrémité 12, proximale, sensiblement cylindrique et percée d'un logement cylindrique 13 sensiblement suivant l'axe correspondant à celui de la portion d'os remplacée. La seconde extrémité 12 porte aussi deux encoches 14, 15 dont une face est perpendiculaire et deux faces sont sensiblement parallèles à l'axe du logement cylindrique 13 ;
- une pièce de transport 2 avec une première extrémité 21 cylindrique dimensionnée de façon à être apte à coulisser dans le logement cylindrique 13 de la pièce de référence 1 et qui comporte suivant son axe :
   o deux premier et second lamages 23, 24 et un perçage 25, successifs, de diamètres décroissants et qui débouchent du coté de ladite première extrémité 21 et
   o deux gorges longitudinales oblongues 26, 27 positionnées de manière à ce que les encoches 14, 15 de la pièce de référence 1 soient toujours en en vis-à-vis quand la pièce de transport 2 est assemblée quelle que soit la phase d'allongement du fémur prothétique. La seconde extrémité 22 de la pièce de transport 2 est en forme de queue qui peut, de manière connue, être cimentée ou enfoncée en force dans le canal médullaire de la portion d'os résiduel de l'os à reconstruire ;
- une tige filetée 3 avec une première extrémité 31 et une seconde extrémité 32 ;
- une barre 34 dite première butée à section sensiblement rectangulaire, dont les extrémités 342, 343 sont aptes à coopérer avec les deux encoches, respectivement 14, 15, pratiquées à la seconde extrémité 12 de la pièce de référence 1, encoches 14, 15 qui constituent le premier appui. Cette barre 34 comporte également sensiblement en son milieu un taraudage 341 apte à recevoir la seconde extrémité 32 de la tige filetée 3, et peut comporter un renflement de renfort 344 autour de ce taraudage 341 ou présenter une épaisseur constante dans la direction perpendiculaire à la tige filetée 3 suffisante pour assurer la résistance aux charges subies ;
- un écrou 4 dont le taraudage intérieur est apte à coopérer avec la tige filetée 3, dont l'extérieur 73 est de préférence cylindrique mais peut aussi être hexagonal par exemple et qui comporte une première face d'extrémité 41 et une seconde face d'extrémité 42 ainsi qu'un épaulement 74 proche de sa seconde extrémité 42 ;
- une première rondelle-palier 51 percée en son centre d'un trou 511 de diamètre juste supérieur à celui de la tige filetée 3 et qui prend appui à la périphérie d'une de ses faces au fond du second lamage 24 de la pièce de transport 2 et coopère sur le pourtour de son trou 511 sur son autre face avec la seconde face d'extrémité 42 de l'écrou 4 pour constituer ensembles les moyens 42, 51 pour transformer le déplacement dans un premier sens de l'écrou 4 le long de la tige filetée 3 en déplacement de la pièce de transport 2 par rapport à la pièce de référence 1 ;
- un aimant 6 en néodyme fer bore résistant à la température de stérilisation, sensiblement cylindrique et percé suivant son axe d'un trou 61 dans lequel peut être introduit et maintenu l'écrou 4, par exemple par collage au moyen d'une colle silicone, cet aimant 6 constituant l'arbre de commande ;
- un capot 71 en forme de segment de tube fermé à une extrémité par une paroi plane , à l'exception d'une ouverture 711 qui s'adapte sur l'épaulement 74 de l'écrou 4, le capot 71 étant fermé, à son autre extrémité, par une rondelle 72 dont le périmètre extérieur s'adapte pour la boucher à l'extrémité ouverte du capot 71, le perçage central 711 laissant juste passer l'extrémité 41 de l'écrou 4, le capot 71 et la rondelle 72 formant, avec la face externe 73 de l'écrou 4, une enceinte isolant l'aimant 6 du milieu biologique, le capot 71 et la rondelle 72 formant avec la colle qui maintient l'aimant 6 dans cette enceinte, les moyens d'entraînement 71, 72 qui relient l'arbre de commande 6 à l'écrou 4, la colle étant de préférence une colle silicone bien tolérée par l'organisme. La face externe 73 de l'écrou 4 et le perçage central 61 de l'aimant 6 peuvent aussi présenter une section qui les bloque en rotation l'une par rapport à l'autre telle qu'une section hexagonale.
- une seconde rondelle-palier 52 percée en son centre d'un trou 521 de diamètre juste supérieur à celui de la tige filetée 3 et qui prend appui à la périphérie d'une de ses faces au fond du premier lamage 23 de la pièce de transport 2 et coopère sur le pourtour de son trou 521 sur cette même face avec la première face d'extrémité 41 de l'écrou 4 pour constituer ensembles les moyens 52, 41 pour transformer le déplacement dans le second sens opposé au premier sens de l'écrou 4 le long de la tige filetée 3 en déplacement de la pièce de transport 2 par rapport à la pièce de référence 1. Cette seconde rondelle-palier 52 peut éventuellement être omise si seul le fonctionnement dans le sens de l'allongement est requis.

Le premier lamage 23 de la pièce de transport 2 est dimensionné pour recevoir sans jeu cette seconde rondelle-pallier 52.

Le second lamage 24 de la pièce de transport 2 est dimensionné pour recevoir outre la première rondelle-palier 51, l'aimant 6 et son enceinte 71, 72, 73 avec un jeu suffisant pour permettre la rotation de ce dernier ensemble.

Le perçage 25 de la pièce de transport 2 est dimensionné de façon à ce que la première butée 34, si elle comporte un renflement de renfort 344, puisse y être introduite, sa plus grande longueur sensiblement suivant l'axe dudit perçage 25, puis, pivotée dans sa position de fonctionnement à travers les gorges longitudinales oblongues 26, 27 de la pièce de transport 2, l'épaisseur de la première butée 34 étant juste inférieure à la largeur des gorges 26, 27 de manière à pouvoir y coulisser à l'exception du renflement 344. Dans une variante de réalisation, le renflement de renfort 344 est absent et la barre 34 formant première butée présente une épaisseur constante suffisante pour résister aux charges prévues, le perçage 25, qui n'a alors plus d'utilité est omis et les gorges 26, 27 confondues en une seule de largeur juste supérieure à l'épaisseur de la barre 34 et qui suffit également au passage de la tige filetée 3 après que la barre 34 ait été introduite par un des cotés débouchant de cette gorge unique.

Le fémur distal suivant le premier mode de réalisation est assemblé de la manière suivante :
- l'écrou 4 et le capot 71 sont assemblés et soudés entre eux à la jonction entre l'épaulement 74 de l'écrou 4 et l'ouverture correspondante 711 du capot 71. Le logement ainsi constitué est encollé par exemple avec une colle silicone bien tolérée par l'organisme et l'aimant 6 y est introduit en pressant de manière à extruder la colle tout autour. La rondelle 72 est rapportée et soudée à sa périphérie sur le capot 71, ce qui clos l'enceinte de l'aimant 6 ;
- la première butée 34 est introduite dans le perçage 25 de la pièce de transport 2 puis pivotée à travers les gorges longitudinales oblongues 26, 27 de manière à la mettre dans sa position de fonctionnement normal, ses faces aptes à coopérer avec le premier appui 14, 15 tournées vers la première extrémité 21 alésée de la pièce de transport 2 ;
- la tige filetée 3 est vissée dans le taraudage 341 de la première butée 34 et soudée en bout au laser sur celle-ci au travers d'une des gorges oblongues longitudinales 26, 27 ;
- la rondelle d'appui 51 est enfoncée jusqu'en butée sur le fond plat du second lamage 24, puis l'écrou 4 est vissé sur la tige filetée 3 et enfin la rondelle-pallier 52 prend place dans le premier lamage 23, de plus grand diamètre, et est soudée en périphérie ;
- l'ensemble ainsi constitué est enfin introduit dans le logement cylindrique 13 de la pièce de référence 1 pour constituer le fémur distal à croissance suivant le première mode de réalisation.

Le fémur distal suivant le premier mode de réalisation peut être allongé de la manière suivante :
- l'arbre de commande 6 est entrainé en rotation dans le sens de l'allongement, qui est celui dans lequel l'écrou 4 se déplace le long de la tige filetée 3 vers la première butée 34 et qui dépend du sens du pas de la tige filetée 3, à l'aide d'un champ magnétique tournant créé par une source de champ externe
   - qui est avantageusement un aimant permanent manipulé à la main et que l'on fait tourner autour de la jambe en maintenant un de ses pôles, par exemple le pôle nord, constamment tourné vers l'aimant 6
   - ou bien, quand il n'est pas possible de tourner sur 360° à distance raisonnable à la hauteur de l'aimant 6 comme au niveau de la hanche par exemple, auquel on fait effectuer alternativement un demi-tour autour de la jambe avec le pôle nord tourné vers l'aimant 6 puis un autre demi-tour autour de la jambe avec le pôle sud tourné vers l'aimant 6, les deux demi-tours étant débutés et achevés aux mêmes endroits. Le pas de la tige filetée 3 étant typiquement compris entre 0.1 et 0.2 mm, cinq à dix tours ou dix à vingt demi-tours produiront un allongement de 1 mm. Plusieurs dispositifs mécaniques ou électromécaniques aptes à entraîner un aimant permanent, ou électromagnétiques connus peuvent aussi créer un tel champ tournant.
- La rotation de l'arbre de commande 6 entraîne celle de l'écrou 4 par l'intermédiaire des moyens d'entraînement 71, 72, 73 reliant l'arbre de commande 6 à l'écrou 4 et la translation vers la première butée 34 de l'écrou 4 le long de la tige filetée 3. La tige filetée 3 prenant appui sur la pièce de référence 1 par l'intermédiaire de la première butée 34 et du premier appui 14, 15, l'écrou 4 se translate aussi par rapport à la pièce de référence 1 et la pièce de transport 2 est ainsi éloignée de la pièce de référence 1 par l'intermédiaire des moyens 51, 42 pour transformer le déplacement dans un premier sens de l'écrou 4 le long de la tige filetée 3 en déplacement de la pièce de transport 2 par rapport à la pièce de référence 1.

La rotation de l'arbre de commande 6 dans un premier sens entraîne donc l'allongement du fémur suivant le premier mode de réalisation, la rotation de l'arbre de commande 6 dans le sens opposé entraîne son raccourcissement mais seulement quand des forces externes, telles que la tension des tissus mous et le poids du patient, aptes à pousser la première butée 34 contre le premier appui 14,15 s'exercent.

Ainsi, dans le premier mode de réalisation :
- les premières extrémités 21, 31 de la pièce de transport 2 et de la tige filetée 3 sont constamment du coté de la première extrémité 11 de la pièce de référence 1 par rapport à sa seconde extrémité 12, quelle que soit la phase d'allongement du dispositif;
- la première butée 34 et l'écrou 4 sont disposés de part et d'autre du premier appui 14, 15,
- la première butée 34 qui coopère avec les encoches 14, 15, par l'intermédiaire de leurs faces perpendiculaires à l'axe du logement cylindrique 13, pour limiter la translation longitudinale de la tige filetée 3 par rapport à la pièce de référence 1 coopère également avec les encoches 14, 15, par l'intermédiaire de leurs faces sensiblement parallèles à l'axe du logement cylindrique 13, pour bloquer la rotation de la tige filetée 3 par rapport à la pièce de référence 1 ;
- l'écrou 4 est libre de tourner par rapport à la pièce de référence 1 ;
- la première butée 34 coopère également avec les gorges longitudinales oblongues 26, 27 de la pièce de transport 2 pour assurer le guidage en rotation et la limitation en translation de cette dernière par rapport à la pièce de référence 1.

On se réfère maintenant aux figures 4 et 5 qui représentent un second mode de réalisation bien adapté à la réalisation de prothèses de croissance, mais également de clous d'allongement ou de transport osseux et de tiges pour la correction du rachis ou du thorax. Pour cet exemple démonstratif mais nullement limitatif du second mode de réalisation, c'est à nouveau une prothèse de fémur distal à croissance qui est représentée. Celle-ci est en tout point identique à celle suivant le premier mode de réalisation sauf que la tige filetée 3 se prolonge du coté de sa première extrémité 31 à travers un perçage 17 pratiqué dans la pièce de référence 1 jusqu'à déboucher à la première extrémité 11 de cette dernière dans un lamage dont le fond constitue un second appui 16 apte à recevoir une seconde butée 33 solidarisée, par exemple par vissage et soudage, à la première extrémité 31 de la tige filetée 3 pour bloquer sa translation longitudinale par rapport à la pièce de référence 1, cette fois dans le sens premier appui 14, 15 vers première butée 34.

La figure 4 montre une vue en perspective de la prothèse de fémur distal à croissance suivant le second mode de réalisation dans laquelle la position de la seconde butée 33 est bien visible.

La figure 5 montre cette prothèse en coupe avant allongement.

Le montage de ce fémur distal à croissance suivant le second mode de réalisation commence comme celui du premier mode de réalisation mais, une fois la pièce de transport 2 introduite dans le logement cylindrique 13 de la pièce de référence 1, se poursuit et se finit par le vissage et la soudure en bout de la seconde butée 33 sur la première extrémité 31 de la tige filetée 3.

Le fémur distal à croissance suivant le second mode de réalisation fonctionne comme celui suivant le premier mode de réalisation mais pourra être raccourci même en l'absence de force externes aptes à pousser la première butée 34 contre le premier appui 14,15 et pourra même produire un effort de compression sur l'organisme, ce qui constitue un avantage par rapport au premier mode de réalisation.

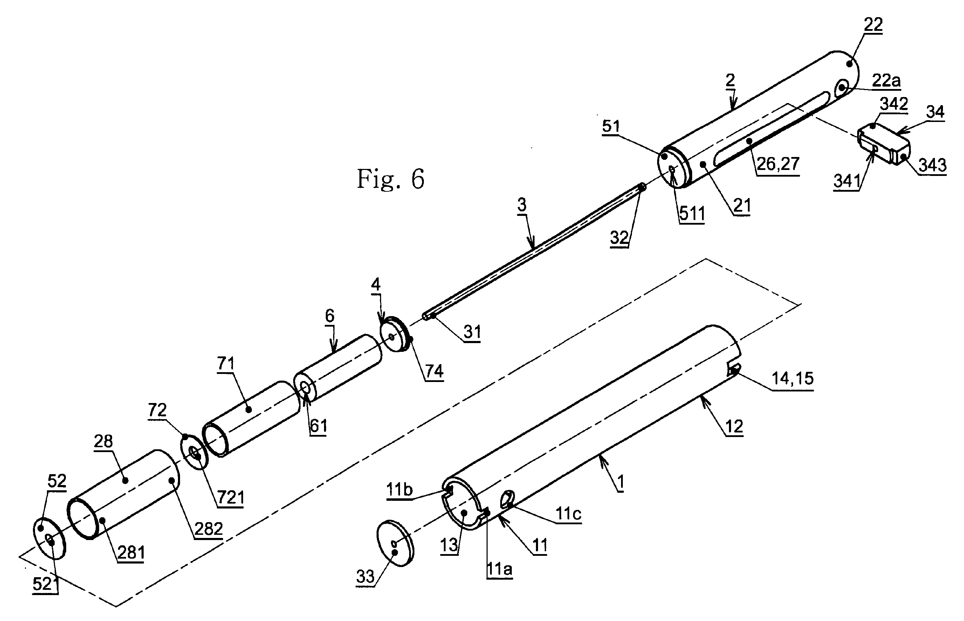

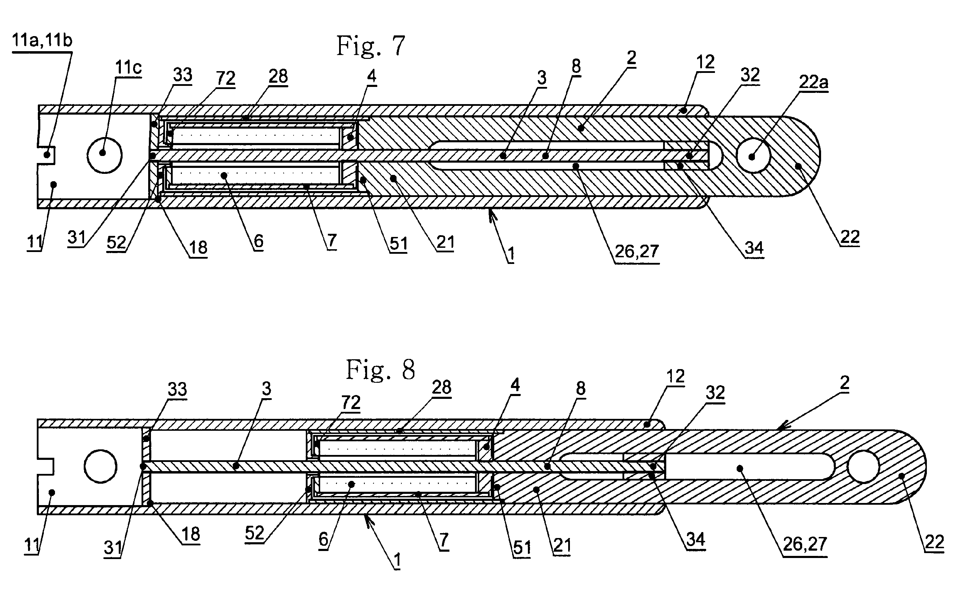

On se réfère maintenant aux figures 6 à 8 qui illustrent un troisième mode de réalisation.

Le dispositif représenté en figures 6 à 8 comprend une pièce de référence 1 tubulaire et sensiblement cylindrique pourvue d'une première extrémité 11 et d'une seconde extrémité 12.

La première extrémité 11 de la pièce de référence 1 comprend des moyens de fixation du matériel ancillaire utilisé pour manipuler le clou pendant son implantation et son explantation. Dans le mode de réalisation représenté, ces moyens comprennent deux encoches 11 a, 11 b, en regard l'une de l'autre, et un taraudage interne à la paroi tubulaire formant la pièce de référence 1. La première extrémité 11 de la pièce de référence 1 comprend également des moyens de fixation à l'organisme. Dans le mode de réalisation représenté, ces moyens de fixation comprennent un trou 11 c de passage d'une vis de fixation, qui peut être lisse ou taraudé.

La seconde extrémité 12 de la pièce de référence 1 comprend deux encoches 14, 15, dites premier appui, encoches dont une face est sensiblement perpendiculaire et deux faces sont sensiblement parallèles à l'axe d'élancement de la pièce de référence 1.

En rappel aux éléments décrits et référencés en figures 1 à 5, le dispositif représenté en figures 6 à 8 comprend une pièce de transport 2, une tige filetée 3, une barre 34 dite première butée, un écrou 4, un aimant 6, un capot 71, 51 et une seconde rondelle palier 52.

La pièce de transport 2 comprend une première extrémité 21 cylindrique dimensionnée de façon à être apte à coulisser dans le logement cylindrique 13 de la pièce de référence 1. La pièce de transport 2 comporte deux gorges longitudinales oblongues 26, 27 positionnées de manière à ce que les encoches 14, 15 de la seconde extrémité 12 de la pièce de référence 1 soient toujours en vis-à-vis quand la pièce de transport 2 est assemblée, quelle que soit la phase d'allongement du dispositif. La seconde extrémité 22 de la pièce de transport 2 est pourvue de moyens de fixation à l'organisme et peut avoir un diamètre égal ou supérieur à celui de la première extrémité 21. Dans le mode de réalisation représenté, ces moyens de fixation comprennent un trou 22a, qui peut être lisse ou taraudé, de passage d'une vis de fixation.

La barre 34 dite première butée comporte deux extrémités 342, 343 radiales aptes à coopérer avec les encoches 14, 15 de la seconde extrémité 12 de la pièce de référence 1. La barre 34 comporte sensiblement en son milieu un logement, préférablement taraudé 341 apte à recevoir la seconde extrémité 32 de la tige filetée 3.

L'écrou 4 est pourvu d'un taraudage intérieur apte à coopérer avec la tige filetée 3.

La première rondelle palier 51 est usinée dans la masse de la pièce de transport 2 et est pourvue en son centre d'un trou 511 de diamètre juste supérieur à celui de la tige filetée 3.

L'aimant 6, avantageusement en néodyme fer bore, résistant à la température de stérilisation, est sensiblement cylindrique et percé suivant son axe d'un trou 61 traversé par la tige filetée 3. L'aimant 6 est logé dans une enceinte formée par le capot 71, une rondelle 72 et l'écrou 4. Le capot 71 est en effet en forme de segment de tube et est fermé à une extrémité par la rondelle 72 et à son autre extrémité par l'écrou 4. La rondelle 72 est pourvu d'un trou 721 de passage de la tige filetée 3 et sert de palier pour la rotation de l'aimant.

Le dispositif selon le troisième mode de réalisation représenté en figures 6 à 8 peut être assemblé de la manière suivante.

L'écrou 4 et le capot 71 sont assemblés et soudés entre eux à la jonction entre l'épaulement 74 de l'écrou 4 et le bord annulaire du capot 71 tubulaire. Le logement ainsi formé est encollé, avantageusement à l'aide d'une colle silicone bien tolérée par l'organisme, et l'aimant 6 est introduit dans le logement encollé en pressant de sorte à extruder la colle tout autour. Pendant cette phase un noyau amovible protège de la colle le trou 61 de l'aimant 6. La rondelle 72 et le capot 71 sont assemblés et soudés entre eux à la jonction entre un épaulement annulaire de la rondelle 72 et le bord annulaire du capot 71 tubulaire.

La première butée 34 est introduite dans la pièce de transport 2 au travers des gorges longitudinales oblongues 26, 27. La tige filetée 3 est introduite dans la pièce de transport 2, au travers du trou 511 de la première rondelle palier 51, et placée ou vissée dans le logement 341 de la première butée 34 puis soudée au laser en bout sur cette première butée 34, au travers d'une des gorges longitudinales oblongues 26, 27.

L'ensemble formé par l'écrou 4, le capot 71, la rondelle 72 et l'aimant 6 est assemblé à la tige filetée 3, par vissage de l'écrou 4 sur la tige filetée 3.

Un élément tubulaire 28 est assemblé et soudé à sa première extrémité 281 à la deuxième rondelle palier 52 pour former une chemise. Cette chemise loge l'arbre de commande formé par l'aimant 6 et son enceinte 71, 72, 4. A sa deuxième extrémité 282, cette chemise est assemblée et soudée à la première extrémité 21 de la pièce de transport 2 sur la rondelle palier 51. La tige filetée 3 débouche, au travers de du trou 521 de la deuxième rondelle palier 52.

L'ensemble ainsi formé est introduit dans la seconde extrémité 12 de la pièce de référence 1, la première butée 34 venant se loger dans les encoches 14, 15 de la pièce de référence. La pièce de référence 1 est pourvue d'un lamage 13 dont le fond constitue un second appui apte à recevoir une seconde butée 33. La première extrémité 31 de la tige filetée 3 est solidarisée par soudage à cette seconde butée 33.

Le dispositif selon le troisième mode de réalisation représenté en figures 6 à 8 peut être allongé de la manière suivante. La seconde extrémité 22 de la pièce de transport 2 et la première extrémité 11 de la pièce de référence 1 étant fixées, notamment au moyens de vis de fixation osseuse au travers des trous 11c, 22a, un champ magnétique tournant est créé par une source de champ externe.

Avantageusement, la source de champ magnétique externe est un aimant permanent manipulé à la main et que l'on fait tourner autour du dispositif en maintenant l'un des pôles de l'aimant, par exemple le pôle nord, constamment tourné vers l'aimant 6. En variante, on fait effectuer alternativement un demi tour de l'aimant permanent autour du dispositif avec le pôle nord tourné vers l'aimant 6 puis un autre demi tour avec le pôle sud tourné vers l'aimant, les deux demi tours étant débutés et achevés aux mêmes endroits.

Le pas de la tige filetée 3 étant par exemple compris entre 0.1 et 0.2 mm, cinq à dix tours ou dix à vingt demi tours produiront un allongement de un millimètre.

Dans d'autres modes de mise en oeuvre, le champ magnétique externe est obtenu à l'aide de dispositifs électromagnétiques.

La rotation de l'aimant 6 dans un premier sens entraîne la rotation de l'écrou 4 sur la vis filetée 3, c'est-à-dire le déplacement de l'écrou 4 vers la première butée 34, provoquant le déplacement de la pièce de transport 2 par rapport à la pièce de référence 1 et un allongement du dispositif. La tige filetée 3 travaille en traction et sa longueur chargée 8 diminue lorsque l'écrou 4 se rapproche de la première butée 34 et que le dispositif s'allonge.

La rotation de l'aimant 6 dans le second sens entraîne le raccourcissement du dispositif lorsque les forces externes s'exercent, telles que celles de la tension des tissus mous et le poids du patient. Dans chacun des trois modes de réalisation, le dispositif comprend une pièce de référence 1, une pièce de transport 2, une tige filetée 3, un écrou 4 monté sur la tige filetée 3, un arbre de commande comprenant un aimant permanent 6, et des moyens d'entraînement 71, 72, 73 reliant l'arbre de commande à l'écrou 4. L'écrou 4 est libre de tourner par rapport à la pièce de référence 1, et l'écrou 4 se déplace le long de la tige filetée 3, la pièce de transport 2 est coulissante par rapport à la pièce de référence 1. La rotation de la tige filetée 3 par rapport à la pièce de référence 1 est bloquée par une première butée 34 coopérant avec un premier appui 14, 15, la première butée 34 étant solidaire d'une extrémité 32 de la tige filetée 3, le premier appui 14, 15 étant solidaire d'une extrémité 12 de la pièce de référence 1 , la première butée 34 et l'écrou 4 étant disposés de part et d'autre du premier appui 14, 15. La translation longitudinale de la tige filetée 3 par rapport à la pièce de référence 1 dans le sens première butée 34 vers premier appui 14 est limitée par la coopération de la première butée 34 avec le premier appui 14.

Les moyens assurant le blocage de la translation de la tige par rapport à la pièce de référence assurent également le blocage de la rotation de la tige filetée par rapport à cette pièce de référence.

La tige filetée est bloquée en rotation par rapport à la pièce de référence par des moyens qui limitent la rotation de la pièce de transport par rapport à la pièce de référence.

Le dispositif selon l'invention, s'il conserve les avantages des dispositifs conçus par l'inventeur, dans lesquels la tige filetée ne travaille qu'en traction et dans lesquels la longueur chargée de la tige filetée diminue quand le dispositif s'allonge, se démarque nettement de ces dispositifs antérieurs. Ainsi, dans le dispositif décrit par le document FR 2901991 de l'inventeur, la tige filetée est entraînée en rotation dans la première pièce et aucun moyen ne bloque sa rotation par rapport à cette première pièce ou par rapport à la deuxième pièce : la pièce référencée 55 dans ce document antérieur, dite languette d'appui, comporte un perçage dans lequel la tige filetée tourne librement.

Dans le dispositif décrit par le document WO 2001/78614, la rotation de l'aimant entraîne la rotation de la tige filetée, provoquant l'allongement du dispositif.

A titre indicatif, dans un dispositif selon l'invention, le diamètre de la tige filetée est de l'ordre de 1,2 à 3 mm, le diamètre extérieur de l'aimant permanent étant compris entre 4 et 12 mm, la longueur de l'aimant permanent étant comprise entre 10 et 100 mm.

Pour illustrer l'invention, on a choisit de représenter en figures 1 à 5 un fémur distal qui est la localisation la plus fréquente dans la pratique clinique mais toute autre prothèses de croissance pour les os longs est réalisable de la même manière en adaptant simplement les diamètres et les longueurs du mécanisme suivant l'invention ainsi que la géométrie des parties en contact avec l'organisme à la localisation envisagée. Par exemple pour réaliser un fémur proximal, la pièce de référence se terminera par une articulation de la hanche, la pièce de transport n'étant pas sensiblement modifiée par rapport au fémur distal ; pour un fémur entier la pièce de transport se terminera par une articulation de la hanche, celle de référence étant inchangée par rapport au fémur distal. De même on réalisera notamment tibia et humérus proximaux prothétiques mais des prothèses à croissance de tous les os longs, y compris des plus petits, tels que le radius ou le cubitus, ou même une phalange sont réalisables suivant la présente invention.

De même, des clous, placés dans le canal médullaire d'un os longs, ou des plaques, placées le long d'un os, pour la chirurgie maxillo-faciale ou celle du bassin notamment, d'allongement et de transport osseux pourront également être réalisés suivant l'invention, en adaptant les longueurs et les diamètres du mécanisme à la localisation du dispositif suivant l'invention, la forme extérieure de la pièce de transport et de celle de référence, qui deviennent sensiblement cylindriques dans la cas d'un clou, comme représenté en figures 6 à 8, avec un coude dans le cas d'un clou tibial par exemple, et plus aplaties dans le cas d'une plaque et sont munies, de manière connue, à leur extrémité libre et, dans le cas des plaques latéralement également, de perçages aptes à recevoir des vis à os.

L'invention s'applique aussi aux matériels pour la correction du rachis et du thorax, tels que tiges de distraction ou de compression qui peuvent être fixées au bassin, aux vertèbres ou aux côtes, en adaptant les longueurs et les diamètres du mécanisme, les extrémités de la pièce de transport et de celle de référence devenant sensiblement cylindriques, comme représenté en figures 6 à 8, et d'un diamètre compatible avec les vis ou les crochets qui servent à les relier au squelette.

On a décrit trois formes préférées de réalisation du dispositif selon l'invention mais d'autres formes de réalisation existent qui répondent à la définition de l'invention.

Parmi elles, celles dans lesquelles les moyens pour transformer le déplacement dans un premier sens de l'écrou le long de la tige filetée en déplacement de la pièce de transport par rapport à la pièce de référence comprennent des moyens d'amplification de force ou de déplacement tel qu'un levier. L'axe de rotation du levier est par exemple solidaire de la pièce de référence, une première extrémité du levier prend appui sur l'écrou et une seconde extrémité sur la pièce de transport. Le rapport des longueurs première extrémité / axe et axe / seconde extrémité du levier, conditionne le type d'amplification produit.

D'autres formes de réalisation existent aussi dans lesquelles les moyens d'entraînement qui relient l'arbre de commande à l'écrou comprennent un réducteur planétaire à arbre creux par exemple qui permet d'amplifier la force produite par le dispositif.

L'arbre de commande peut en outre comporter un moteur électrique ou un moteur à ressort par exemple.

Le dispositif suivant l'invention est avantageusement réalisé dans des matériaux résistants mécaniquement et bien tolérés par l'organisme, tels que des aciers inoxydables comme le 316L, des alliages de titane, des polymères tels que le Poly-Ether-Ether-Cétone (PEEK) ou, de préférence, comme des alliages hautes performances à base de chrome et de cobalt comme par exemple l'alliage austénitique commercialisé par la société Arcelor Mittal sous l'appellation PHYNOX (désignation AFNOR: K 13C20N16Fe15D07).

Par ailleurs, les surfaces soumises au frottement dudit dispositif, en particulier la tige filetée 3, peuvent avantageusement recevoir un traitement de surface anti-usure et/ou diminuant leur coefficient de frottement à base de carbone amorphe diamantin ou de bisulfure de tungstène par exemple.

Le dispositif suivant l'invention est particulièrement utile notamment pour la réalisation de tiges pour la correction du rachis ou du thorax, de clous et de plaques d'allongement ou de transport osseux, y compris pour la chirurgie maxillo-faciale, et de prothèses à croissance.

Le dispositif selon l'invention trouve également application dans l'allongement ou l'extension ou la déformation des tissus mous tels qu'une partie de l'intestin, ou bien encore pour le cerclage artériel, les anneaux de valvuloplastie à géométrie évolutive, les anneaux gastriques.

## Revendications

1. Dispositif de déplacement de tissus à l'intérieur de l'organisme, notamment de tissus osseux, comprenant une pièce de référence (1), une pièce de transport (2), une tige filetée (3), un écrou (4) monté sur la tige filetée (3), un arbre de commande et des moyens d'entraînement (71, 72, 73) reliant l'arbre de commande à l'écrou (4), la pièce de transport (2) étant montée coulissante par rapport à la pièce de référence (1) et limitée en rotation par rapport à la pièce de référence (1) par une première butée (34) qui coopère avec un premier appui (14, 15), **caractérisé en ce que** la rotation de la tige filetée (3) par rapport à la pièce de référence (1) est bloquée par la première butée (34), l'écrou (4) étant libre de tourner par rapport à la pièce de référence (1).

2. Dispositif selon la revendication 1, **caractérisé en ce que** la première butée (34) est solidaire d'une extrémité (32) de la tige filetée (3), le premier appui (14, 15) étant solidaire d'une extrémité (12) de la pièce de référence (1), la première butée (34) et l'écrou (4) étant disposés de part et d'autre du premier appui (14, 15), la translation longitudinale de la tige filetée (3) par rapport à la pièce de référence (1) dans le sens première butée (34) vers premier appui (14) étant limitée par la coopération de la première butée (34) avec le premier appui (14,15).

3. Dispositif suivant la revendication 1 ou 2, **caractérisé en ce qu'**il comporte une seconde butée (33) solidaire d'une extrémité (31) de la tige filetée (3) et apte à coopérer avec un second appui (16) solidaire de la pièce de référence (1) pour limiter la translation longitudinale de la tige filetée (3) par rapport à la pièce de référence (1) dans le sens premier appui (14, 15) vers première butée (34), lesdits premier (14, 15) et second appuis (16) étant placés entre lesdites première (34) et seconde butées (33) et ledit écrou (4) étant apte à se déplacer le long de la tige filetée (3) entre lesdits premier (14, 15) et second (16) appuis.

4. Dispositif suivant l'une quelconque des revendications 1 à 3 **caractérisé en ce que** l'arbre de commande comprend un aimant permanent (6)

5. Dispositif selon la revendication 4, **caractérisé en ce que** l'aimant permanent (6) est solidaire de l'écrou (4).

6. Dispositif selon la revendication 4 ou 5, **caractérisé en ce que** la direction d'aimantation de l'aimant permanent (6) est sensiblement diamétrale par rapport à l'axe de l'écrou (4).

7. Dispositif suivant la revendication 6, **caractérisé en ce que** l'aimant permanent (6) est un aimant néodyme qui comporte un perçage axial (61) pour laisser passer notamment la tige filetée (3) et que les moyens d'entraînement qui relient l'arbre de commande à l'écrou (4) sont une enveloppe rigide (72, 73, 74) solidaire de l'écrou (4) et dans laquelle est maintenu l'aimant permanent (6).

8. Dispositif suivant l'une quelconque des revendications 1 à 7, **caractérisé en ce qu'**il comprend des moyens pour transformer le déplacement dans un premier sens de l'écrou (4) le long de la tige filetée (3) en déplacement de la pièce de transport (2) par rapport à la pièce de référence (1), ces moyens étant une première surface d'appui (42) solidaire de l'écrou (4) et une seconde surface d'appui (51) solidaire de la pièce de transport (2), aptes à coopérer et sensiblement perpendiculaires à l'axe de la tige filetée (3).

9. Dispositif suivant l'une quelconque des revendications 1 à 8, **caractérisé en ce qu'**il comprend des moyens pour transformer le déplacement dans un second sens de l'écrou (4) le long de la tige filetée (3) en déplacement de la pièce de transport (2) par rapport à la pièce de référence (1), ces moyens étant une troisième surface d'appui (41) solidaire de l'écrou (4) et une quatrième surface d'appui (52) solidaire de la pièce de transport (2), aptes à coopérer et sensiblement perpendiculaires à l'axe de la tige filetée (3).

10. Dispositif selon l'une quelconque des revendications 1 à 9, **caractérisé en ce que** la pièce de référence (1) comporte une articulation prothétique du genou, de la hanche, de l'épaule ou du coude.

## Patentansprüche

1. Vorrichtung zur Verschiebung von Gewebe im Inneren des Organismus, insbesondere Knochengewebe, umfassend ein Bezugsteil (1), ein Transportteil (2), eine Gewindestange (3), eine an der Gewindestange (3) montierte Mutter (4), eine Schaltwelle und Antriebsmittel (71, 72, 73), die die Schaltwelle mit der Mutter (4) verbinden, wobei das Transportteil (2) so montiert ist, dass es bezüglich des Bezugsteils (1) gleitet und bezüglich des Bezugsteils (1) durch einen ersten Anschlag (34) drehbegrenzt ist, der mit einem ersten Auflager (14, 15) zusammenwirkt, **dadurch gekennzeichnet, dass** die Drehung der Gewindestange (3) bezüglich des Bezugsteils (1) durch den ersten Anschlag (34) blockiert ist, wobei sich die Mutter (4) bezüglich des Bezugsteils (1) frei drehen kann.

2. Vorrichtung nach Anspruch 1, **dadurch gekennzeichnet, dass** der erste Anschlag (34) fest mit einem Ende (32) der Gewindestange (3) verbunden ist, wobei das erste Auflager (14, 15) fest mit einem Ende (12) des Bezugsteils (1) verbunden ist, wobei der erste Anschlag (34) und die Mutter (4) beidseitig des ersten Auflagers (14, 15) angeordnet sind, wobei die Längstranslation der Gewindestange (3) bezüglich des Bezugsteils (1) in der Richtung erster Anschlag (34) zum ersten Auflager (14) durch das Zusammenwirken des ersten Anschlags (34) mit dem ersten Auflager (14, 15) begrenzt ist.

3. Vorrichtung nach Anspruch 1 oder 2, **dadurch gekennzeichnet, dass** sie einen zweiten Anschlag (33) umfasst, der fest mit einem Ende (31) der Gewindestange (3) verbunden und geeignet ist, mit einem zweiten Auflager (16) zusammenzuwirken, das fest mit dem Bezugsteil (1) verbunden ist, um die Längstranslation der Gewindestange (3) bezüglich des Bezugsteils (1) in der Richtung erstes Auflager (14, 15) zum ersten Anschlag (34) zu begrenzen, wobei das erste (14, 15) und das zweite Auflager (16) zwischen dem ersten (34) und dem zweiten Anschlag (33) platziert sind und die Mutter (4) geeignet ist, sich entlang der Gewindestange (3) zwischen dem ersten (14, 15) und dem zweiten Auflager (16) zu verschieben.

4. Vorrichtung nach einem der Ansprüche 1 bis 3, **dadurch gekennzeichnet, dass** die Schaltwelle einen Permanentmagneten (6) umfasst.

5. Vorrichtung nach Anspruch 4, **dadurch gekennzeichnet, dass** der Permanentmagnet (6) fest mit der Mutter (4) verbunden ist.

6. Vorrichtung nach Anspruch 4 oder 5, **dadurch gekennzeichnet, dass** die Magnetisierungsrichtung des Permanentmagneten (6) im Wesentlichen diametral zur Achse der Mutter (4) verläuft.

7. Vorrichtung nach Anspruch 6, **dadurch gekennzeichnet, dass** der Permanentmagnet (6) ein Neodym-Magnet ist, der eine Axialbohrung (61) umfasst, damit insbesondere die Gewindestange (3) hindurchgehen kann, und dass die Antriebsmittel, die die Schaltwelle mit der Mutter (4) verbinden, eine starre Hülle (72, 73, 74) sind, die fest mit der Mutter (4) verbunden ist und in der der Permanentmagnet (6) gehalten ist.

8. Vorrichtung nach einem der Ansprüche 1 bis 7, **dadurch gekennzeichnet, dass** sie Mittel zur Umwandlung der Verschiebung in einer ersten Richtung der Mutter (4) entlang der Gewindestange (3) in eine Verschiebung des Transportteils (2) bezüglich des Bezugsteils (1) umfasst, wobei diese Mittel eine fest mit der Mutter (4) verbundene erste Auflagerfläche (42) und eine fest mit dem Transportteil (2) verbundene zweite Auflagerfläche (51) sind, die zusammenwirken können und im Wesentlichen senkrecht zu der Achse der Gewindestange (3) verlaufen.

9. Vorrichtung nach einem der Ansprüche 1 bis 8, **dadurch gekennzeichnet, dass** sie Mittel zur Umwandlung der Verschiebung in einer zweiten Richtung der Mutter (4) entlang der Gewindestange (3) in eine Verschiebung des Transportteils (2) bezüglich des Bezugsteils (1) umfasst, wobei diese Mittel eine fest mit der Mutter (4) verbundene dritte Auflagerfläche (41) und eine fest mit dem Transportteil (2) verbundene vierte Auflagerfläche (52) sind, die zusammenwirken können und im Wesentlichen senkrecht zu der Achse der Gewindestange (3) verlaufen.

10. Vorrichtung nach einem der Ansprüche 1 bis 9, **dadurch gekennzeichnet, dass** das Bezugsteil (1) eine Knie-, Hüft-, Schulter- oder Ellenbogengelenkprothese umfasst.

## Claims

1. Device for moving tissues inside the body, in particular bone tissues, comprising an index part (1), a transport part (2), a threaded rod (3), a nut (4) mounted on the threaded rod (3), a control shaft, and drive means (71, 72, 73) linking the control shaft to the nut (4), the transport part (2) being mounted slidably with respect to the index part (1) and being limited in rotation with respect to the index part (1) by a first stop (34) which cooperates with a first bearing (14, 15), **characterized in that** the rotation of the threaded rod (3) with respect to the index part (1) is blocked by the first stop (34), the nut (4) being free to turn with respect to the index part (1).

2. Device according to Claim 1, **characterized in that** the first stop (34) is rigidly connected to an end (32) of the threaded rod (3), the first bearing (14, 15) being rigidly connected to an end (12) of the index part (1), the first stop (34) and the nut (4) being arranged on both sides of the first bearing (14, 15), the longitudinal translation of the threaded rod (3) with respect to the index part (1) in the sense of first stop (34) to first bearing (14) being limited by the cooperation of the first stop (34) with the first bearing (14, 15).

3. Device according to Claim 1 or 2, **characterized in that** it has a second stop (33) rigidly connected to an end (31) of the threaded rod (3) and capable of cooperating with a second bearing (16) rigidly connected to the index part (1) for limiting the longitudinal translation of the threaded rod (3) with respect to the index part (1) in the sense of first bearing (14, 15) to first stop (34), said first bearing (14, 15) and second bearing (16) being placed between said first stop (34) and second stop (33), and said nut (4) being capable of moving along the threaded rod (3) between said first bearing (14, 15) and second bearing (16).

4. Device according to any one of Claims 1 to 3, **characterized in that** the control shaft comprises a permanent magnet (6).

5. Device according to Claim 4, **characterized in that** the permanent magnet (6) is rigidly connected to the nut (4).

6. Device according to Claim 4 or 5, **characterized in that** the direction of magnetization of the permanent magnet (6) is substantially diametral with respect to the axis of the nut (4).

7. Device according to Claim 6, **characterized in that** the permanent magnet (6) is a neodymium magnet that has an axial hole (61) allowing passage of the threaded rod (3) in particular, and **in that** the drive means linking the control shaft to the nut (4) are in the form of a rigid envelope (72, 73, 74) which is rigidly connected to the nut (4) and in which the permanent magnet (6) is held.

8. Device according to any one of Claims 1 to 7, **characterized in that** it comprises means for transforming the movement of the nut (4) in a first direction along the threaded rod (3) into a movement of the transport part (2) with respect to the index part (1), these means being a first bearing surface (42) rigidly connected to the nut (4) and a second bearing surface (51) rigidly connected to the transport part (2), which are capable of cooperating and are substantially perpendicular to the axis of the threaded rod (3).

9. Device according to any one of Claims 1 to 8, **characterized in that** it comprises means for transforming the movement of the nut (4) in a second direction along the threaded rod (3) into a movement of the transport part (2) with respect to the index part (1), these means being a third bearing surface (41) rigidly connected to the nut (4) and a fourth bearing surface (52) rigidly connected to the transport part (2), which are capable of cooperating and are substantially perpendicular to the axis of the threaded rod (3).

10. Device according to any one of Claims 1 to 9, **characterized in that** the index part (1) has a prosthetic knee, hip, shoulder or elbow joint.
